(19) Europäisches Patentamt
European Patent Office
Office européen des brevets

(11) EP 0 973 033 B1

(12) # EUROPÄISCHE PATENTSCHRIFT

(45) Veröffentlichungstag und Bekanntmachung des Hinweises auf die Patenterteilung:
**28.08.2002 Patentblatt 2002/35**

(51) Int Cl.$^7$: **G01N 33/52**, G01N 33/84, G01N 21/64, G01N 21/77

(21) Anmeldenummer: **99890216.7**

(22) Anmeldetag: **30.06.1999**

(54) **Ionensensor**

Ion sensor

Senseur ionique

(84) Benannte Vertragsstaaten:
**DE FR GB**

(30) Priorität: **30.06.1998 AT 113898**

(43) Veröffentlichungstag der Anmeldung:
**19.01.2000 Patentblatt 2000/03**

(73) Patentinhaber: **F. HOFFMANN-LA ROCHE AG**
**4070 Basel (CH)**

(72) Erfinder:
- **Krause, Christian**
  **84109 Woerth/Isar (DE)**
- **Huber, Christian**
  **93326 Abensberg (DE)**
- **Leiner, Marco Jean Pierre**
  **8045 Graz (AT)**
- **Werner, Tobias**
  **93051 Regensburg (DE)**
- **Wolfbeis, Otto S.**
  **93051 Regensburg (DE)**

(74) Vertreter: **Schwarz, Albin, Dr. et al**
**Patentanwalt**
**Wipplingerstrasse 32/22**
**1010 Wien (AT)**

(56) Entgegenhaltungen:
**EP-A- 0 358 991**
**EP-A- 0 484 865**
**WO-A-93/12428**
**WO-A-95/26501**
**US-A- 4 557 900**
**US-A- 4 645 744**
**US-A- 4 649 123**
**US-A- 5 211 914**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 973 033 B1

## Beschreibung

Die vorliegende Erfindung betrifft einen optisch-chemischen Sensor (Optode) mit einem hydrophilen Bereich und einem hydrophoben Bereich zum Bestimmen von Ionen in einer wässerigen Probe nach dem Koextraktionsprinzip, gemäß welchem das zu bestimmende Ion zunächst aus der Probe in den hydrophilen Bereich wandert und zusammen mit einem optisch detektierbaren, zum bestimmenden Ion elektrisch gegensinnig geladenen Farbstoff (=Luminophor oder Chromophor), der im hydrophilen Bereich vorgesehen ist, in den hydrophoben Bereich, der einen Ionophor für das zu bestimmende Ion enthält, extrahiert wird.

Die Bestimmung der Konzentration ionischer Substanzen findet vielfältige Anwendung in der Analytik. Die einfache und schnelle Bestimmung von $Li^+$, $Na^+$, $K^+$, $Mg^{++}$, $Ca^{++}$ und $Cl^-$, ohne zusätzliche Behandlungsschritte der Probe und unter Ausschluß von Störgrößen, ist vor allem in der medizinischen Diagnostik von besonderer Bedeutung.

Ionenselektive Elektroden werden seit vielen Jahren zur Bestimmung von Kationen und Anionen eingesetzt. Entscheidende Nachteile elektrochemischer Meßanordnungen sind die Notwendigkeit eines Referenzelementes, die Empfindlichkeit gegen elektrische Potentiale und elektromagnetische Störungen.

Optoden hingegen benötigen kein Referenzelement. Die optischen Signale sind unabhängig von externen Potentialen und Stromflüssen. Bisher bekannt gewordene Optoden zur Bestimmung von Ionenkonzentrationen beruhen auf der Messung der direkt oder indirekt von der Aktivität des zu bestimmenden Ions abhängigen Lumineszenzintensität oder Lumineszenzabklingzeit eines Luminophors bzw. der Lichtabsorption eines Chromophors.

Es ist bereits eine Vielzahl von Optoden bekannt, welche auf die angeführten Ionen mit einer Änderung einer oder mehrerer optischer Eigenschaften eines Farbstoffes reagieren. Bisher bekannt gewordene optische Sensoren zur Bestimmung der Konzentration bzw. Aktivität von Ionen in einem Probenmedium beruhen auf unterschiedlichen Verfahren. Beispielsweise beschreiben die US-A - 4,645,744 (Charlton), die US-A - 4,649,123 (Charlton), die EP-B - 0 358 991 (Simon) und die US-A - 5,211,914 (Vogel) optische Sensoren und Meßverfahren zur Bestimmung von Kationen, wobei ein pH-sensitiver, lipophiler Chromophor (Luminophor) und ein ladungsneutraler, lipophiler Ionophor in einer im wesentlichen hydrophoben Polymermatrix vorliegen.

Die vorbekannten Meßverfahren basieren darauf, daß der Ionophor das zu bestimmende Kation komplexiert und in Form eines geladenen Ionophor-Ion-Komplexes in die hydrophobe Polymermatrix extrahiert. Infolge der Ladungsverteilung zwischen Probe und Polymermatrix kommt es zur Bildung eines elektrischen Potentials, welches der weiteren Extraktion entgegenwirkt. Zur Wahrung der Elektroneutralität gibt der in der hydrophoben Polymermatrix befindliche pH-sensitive Luminophor Protonen an die Probe ab. Die Abgabe von Protonen führt zu einer Änderung einer optischen Eigenschaft des Luminophors. Aufgrund dieser Änderung kann auf die Konzentration des zu bestimmenden Ions rückgeschlossen werden. Somit basieren die geoffenbarten Meßverfahren zur Bestimmung von Kationen darauf, daß Kationen reversibel mit dem Probenmedium (beispielsweise $K^+$ gegen $H^+$ oder $Ca^{++}$ gegen $2H^+$) ausgetauscht werden (Ionenaustausch). Infolge des 1:1 Austausches mit Protonen sind die Meßergebnisse in besonders starkem Maß (inhärent) vom pH-Wert der Probe abhängig.

Das europäische Patent EP-B - 0 358 991 und Anal. Chim. Acta. 255, 1991 (Seiten 35 bis 44) beschreiben ähnliche Meßverfahren, jedoch zur Bestimmung von Chlorid. Hier liegen ein anionischer lipophiler, pH-sensitiver Chromophor und eine lipophile, kationische Substanz in einer im wesentlichen hydrophoben Polymermatrix vor. Das geoffenbarte Meßverfahren basiert darauf, daß die kationische Substanz $Cl^-$ und der anionische Chromophor $H^+$ im Verhältnis 1:1 aus dem Probenmedium extrahieren. Durch die Bindung von $H^+$ ändert der Chromophor seine optischen Eigenschaften. Anhand dieser Änderung kann auf die Konzentration des zu bestimmenden Anions rückgeschlossen werden. Somit beruhen die geoffenbarten optischen Meßverfahren zur Bestimmung von Anionen darauf, daß Anionen und Kationen (beispielsweise $Cl^-$ und $H^+$) reversibel aus dem Probenmedium extrahiert werden (Koextraktion). Infolge der Koextraktion von Protonen sind die Meßergebnisse in besonders starkem Maß (inhärent) vom pH-Wert der Probe abhängig.

Derartige Meßverfahren, die in der oben beschriebenen Weise auf Ionenaustausch und Koextraktion beruhen, sind für jene Meßsituationen geeignet, wo der pH-Wert der Probe mit einem pH-Puffer eingestellt werden kann, der pH-Wert der Probe sehr genau bekannt ist oder mittels einer zusätzlichen pH-puffemden Schicht auf einen bekannten Wert eingestellt werden kann.

Bekannt sind auch ionenselektive Membranen, die einen Kaliumionophor in einer hydrophoben Membran enthalten und nach dem Koextraktionsprinzip funktionieren (H. Sumiyoshi, K. Nakahara, Talanta, 24, 763 (1977)). Dabei wird Kalium zusammen mit einem lipophilen Anion (zur Aufrechterhaltung der Elektroneutralität), das der Probe zugegeben wird, in die hydrophobe Membran extrahiert. Falls das Anion gefärbt ist, kann Kalium über die Absorption oder Fluoreszenz des Anions gemessen werden. Derartige optische Meßverfahren sind für jene Meßsituationen geeignet, wo der Probe vor der Messung ein anionischer Chromophor oder Luminophor zugesetzt werden kann.

Ein Sensor der eingangs beschriebenen Art zur Bestimmung von $K^+$ ist aus Anal.Sci. 14, 163ff (1998) bekannt. Bei dieser ionenselektiven Optode liegen ein hinsichtlich Kalium selektiver Ionophor (Valinomycin) gelöst in einer ersten, auf einem optisch transparenten Trägermaterial befindlichen, hydrophoben Schicht bestehend aus weichgemachtem PVC und ein anionischer, pH-insensitiver Luminophor (Sulforhodamin B) in einer zweiten, aus einem hydrophilen Polyurethan bestehenden Schicht vor. Ein Auswandern des Chromophors in die Probe wird durch eine dritte, aus einem Kationentauscher (Nafion) bestehenden Schicht verhindert. Aufgrund der hohen Ansprechzeit von über einer Stunde sind derartig aufgebaute Sensoren insbesondere für klinische Meßsituationen nicht geeignet.

Aufgabe der Erfindung ist es, einen optischen Sensor vorzuschlagen, welcher die oben beschriebenen Beschränkungen nicht aufweist und welcher insbesondere in der medizinischen Diagnostik emöglicht, die Konzentration/ Aktivität von Ionen in Körperflüssigkeiten besonders rasch und effizient zu bestimmen. Ferner soll der Sensor nicht auf einen Farbstoff angewiesen sein, der der Probe zugesetzt werden muß.

Der erfindungsgemäße Sensor besitzt einen hydrophilen Bereich und einen hydrophoben Bereich und dient zum Bestimmen von Ionen in einer wässerigen Probe nach dem Koextraktionsprinzip, gemäß welchem das zu bestimmende Ion zunächst aus der Probe in den hydrophilen Bereich wandert und zusammen mit einem optisch detektierbaren, zum bestimmenden Ion elektrisch gegensinnig geladenen Farbstoff, der im hydrophilen Bereich vorgesehen ist, in den hydrophoben Bereich, der einen Ionophor für das zu bestimmende Ion enthält, extrahiert wird, und ist dadurch gekennzeichnet, daß der hydrophobe Bereich im hydrophilen Bereich eingebettet ist.

Es hat sich gezeigt, daß die Ansprechzeit eines derartig aufgebauten Sensors kurz, insbesondere weniger als eine Minute, ist.

Eine bevorzugte Ausführungsform des erfindungsgemäßen Sensors besteht darin, daß der hydrophobe Bereich in Form von Tröpfchen im hydrophilen Bereich eingebettet ist, wobei als Tröpfchen vorzugsweise ein Weichmacher, z.B. eine hochsiedende organische Flüssigkeit, vorgesehen ist. Die Tröpfchen können durch ein Tensid oder durch Kettensequenzen eines Multi-block-co-polymers stabilisiert sein. Es kann ferner ein Multi-block-co-polymer verwendet werden, welches Kettensegmente mit hydrophilen und hydrophoben Eigenschaften besitzt. Kettensegmente mit hydrophoben Eigenschaften kann man durch Co-Polymerisation von pre-Polymeren (Oligomeren) mit hydrophilen oder hydrophoben Eigenschaften erhalten.

Erfindungsgemäß kann der Sensor auch eine zusätzliche Schicht aufweisen, wobei die zusätzliche Schicht aus einem hydrophilen, ionenpermeablen Polymer aufgebaut ist, welche zwecks optischer Entkopplung von der Probe mit Farbpigmenten, vorzugsweise Schwarzpigmenten, versehen ist.

Bevorzugte Ausführungsformen des erfindungsgemäßen Sensors eignen sich zur Bestimmung von Alkali-, Erdalkali- und Chloridionen.

In einer bevorzugten Ausführungsform des erfindungsgemäßen Sensors ist der hydrophile Bereich eine Schicht, in welche der hydrophobe Bereich in Form von Tröpfchen gebildet ist, die in dieser Schicht emulgiert vorliegen. Diese Ausführungsform ist in der Fig. 1 dargestellt und weist einen transparenten Träger 1 auf, der z.B. als planare Folie oder auch als optische Faser ausgebildet sein kann. Auf dem Träger 1 ist eine Schicht 4 aus einem essentiell hydrophilen, ionenpermeablen Polymer aufgebracht. Als "essentiell hydrophil" wird ein Material verstanden, welches im Gleichgewicht mit Wasser einen Wassergehalt von mindestens 10% aufweist und auch für nicht lipophile Ionen permeabel ist.

Die Schicht 4 enthält fein verteilte Tröpfchen 5 eines essentiell hydrophoben Materiales. Als "essentiell hydrophob" wird ein Polymer verstanden, welches im Gleichgewicht mit Wasser weniger als 10% Wasser aufweist und für nicht lipophile Ionen impermeabel ist.

Der lipophile Ionophor I liegt in den hydrophoben Tröpfchen 5 und der Luminophor $A^-$ im hydrophilen Bereich vor. Als hydrophobe Tröpfchen eignen sich insbesondere hochsiedende organische Substanzen (geeignete Substanzen sind z.B. die weiter unten angeführten Weichmacher). Besonders vorteilhaft sind die Tröpfchen mit vorzugsweise nicht-ionischen Detergentien stabilisiert.

Die zu bestimmenden Ionen $K^+$ wandern aus der Probe in die hydrophile Schicht 4 und werden von dort vom Ionophor I, der sich in den hydrophoben Tröpfchen 5 befindet, in die hydrophoben Tröpfchen 5 extrahiert. Aus Elektroneutralitätsgründen wird pro Ion $K^+$ auch ein Luminophormolekül $A^-$ in die hydrophoben Tröpfchen 5 ko-extrahiert. Durch den Übertritt in die Tröpfchen 5 ändert der Luminophor seine optischen Eigenschaften, was mittels einer Lumineszenzmessung festgestellt wird. Diese Messung ist in der Fig. 1 schematisch mittels des Anregungslichtes hν und des Emissionslichtes hν' angedeutet. Mit der Bezugsziffer 6 ist ein Tröpfchen dargestellt, welches sowohl den Komplex aus Ionophor und zu bestimmendem Ion als auch den Luminophor $A^-$ enthält.

Eine andere, besonders vorteilhafte Ausführungsform eines erfindungsgemäßen Sensors ist in Fig. 2c dargestellt. In dieser Ausführungsform werden der hydrophile und der hydrophobe Bereich in einem Multi-block-co-Polymer gebildet, welches als Schicht 2 auf einem Träger 1 aufgetragen ist, die Tröpfchen 12 aus einem essentiell hydrophoben Material enthält.

Derartige Polymere sind dadurch gekennzeichnet, daß jede Polymerkette aus mehreren Sequenzen von Einheiten mit hydrophilischen Eigenschaften, z.B. unabhängig voneinander Säureamidgruppen, die auch mit hydrophilischen Gruppen substituiert sein können, Säureimidgruppen, Carboxylatgruppen, und mehreren Sequenzen mit Gruppen hoher cohäsiver Energie, z.B. Nitrilgruppen, besteht. Derartige Polymere sind beispielsweise unter dem Warenzeichen HYPAN von der Firma HYMEDIX Int. Inc., Dayton, NJ, USA erhältlich. Die Primärstruktur eines derartigen

Polymers ist in der Fig. 2a dargestellt. Die Herstellung derartiger Polymere ist in der US-A - 4,331,783 und der US-A - 4,379,874 beschrieben.

Beim festen Polymer bilden die hydrophilen Sequenzen durchgehende amorphe ionenpermeable Bereiche (sogenannte weiche Blöcke), während die cohäsiven Sequenzen kristalline Cluster (sogenannte harte Blöcke) ausbilden. Die Fig. 3 zeigt schematisch die Abfolge der weichen Blöcke 10 und der harten Blöcke 11. In der Fig. 2b ist dargestellt, wie sich die cohäsiven Sequenzen einiger Polymerketten zur Bildung der harten Blöcke 8 aneinanderlagern. Mit der Bezugsziffer 9 ist ein weicher Block aus hydrophilen Sequenzen bezeichnet.

Zur Bildung der Schicht 2 wird in das Polymer eine hochsiedende, hydrophobe, niedermolekulare organische Substanz emulgiert. Die Emulsion wird vorzugsweise durch die harten Blöcke stabilisiert. Erfindungsgemäß befindet sich der lipophile Ionophor in den Bereichen mit Weichmacher und der Luminophor in den ionenpermeablen hydrophilen Bereichen. Dies ist in der Fig. 2c dargestellt, welche die Weichmachertröpfchen 12 zeigt, die von den harten Blöcken 8 stabilisiert sind.

Die erfindungsgemäße Anordnung ist somit auch in dieser Ausführungsform verwirklicht: Die zu bestimmenden Ionen $K^+$ wandern aus der Probe in den weichen hydrophilen Bereich 9 und werden von dort vom Ionophor I, der sich in den Tröpfchen 12, stabilisiert durch die harten Kettensequenzen 8, befindet, in die Tröpfchen 12 extrahiert. Aus Elektroneutralitätsgründen wird pro Ion $K^+$ auch ein Luminophormolekül A' in die hydrophoben Tröpfchen 12 ko-extrahiert. Durch den Übertritt in den hydrophoben Bereich 12 ändert der Luminophor seine optischen Eigenschaften, was mittels einer Lumineszenzmessung festgestellt wird.

Beschreibung erfindungsgemäß verwendeter Lumineszenz- und Absorptionsfarbstoffe

Die wichtigste Bedingung an den Farbstoff ist die Ladungszahl. Sie muß gegensätzlich zum Analyten sein, also negativ für Kationen und positiv für Anionen. Des weiteren darf der Farbstoff im pH-Bereich der Messung naturgemäß nicht seine Ladung in Abhängigkeit vom pH-Wert ändern. Seine Lipophilie und seine spektralen Eigenschaften sollten sich mit dem pH-Wert nicht wesentlich ändern.

Weiters muß der Farbstoff zumindest eine optische Eigenschaft aufweisen, die sich beim Übergang vom hydrophilen in den hydrophoben Bereich ändert (z.B. Extinktionskoeffizient, Absorptionsspektrum, Lumineszenzspektrum, Lumineszenzintensität, Lumineszenzquantenausbeute, Lumineszenzabklingzeit). In der Regel erfüllen solvatochrome Farbstoffe zumindest eine dieser Voraussetzungen.

Ein geeigneter Farbstoff braucht eine "ausgewogene" Lipophilie (HLB, "hydrophilicity/lipophilicity balance"), d.h. er darf nicht signifikant von der hydrophilen Sensorphase in die Probe wandern und nicht mit jedem Gegenion aus der hydrophilen Phase in die hydrophobe Phase wandern. Anderseits darf er nicht so hydrophil sein, daß er auch mit dem Analyt nicht in die hydrophobe Phase wandert.

Dieser Sachverhalt kann durch folgende Formeln dargestellt werden:

$$L_s \xleftrightarrow{K_{s/w}} L_w \xleftrightarrow{K_{w/o}} L_o$$

wobei $L_s$ den Farbstoff in der wässerigen Probe, $L_w$ den Farbstoff in der hydrophilen Sensorphase und $L_o$ den Farbstoff in der hydrophoben Sensorphase bedeuten. $K_{s/w}$ steht für das Verteilungsgleichgewicht zwischen der wässerigen Probe und hydrophiler Sensorphase und $K_{w/o}$ für das Verteilungsgleichgewicht zwischen hydrophiler und hydrophober Sensorphase.

Vorzugsweise wird die HLB durch folgende Werte gekennzeichnet:

$$K_{s/w} = \frac{aL_w}{aL_s} \geq 100$$

$$K_{w/o} = \frac{aL_w}{aL_s} \leq 0.25$$

wobei a für die Aktivität des Farbstoffs in der jeweiligen Phase steht.

Da die Hydrophilie bzw. Lipophilie von Farbstoffen durch Einführung hydrophiler bzw. lipophiler chemischer Gruppen verändert werden kann, sind alle solvatochromen Farbstoffe, welche den obigen Anforderungen genügen, prinzipiell einsetzbar. In Abhängigkeit von den optischen Eigenschaften können sowohl Absorptionsfarbstoffe (Transmissions- und Reflektionsmessungen) als auch Lumineszenzfarbstoffe (Lumineszenzintensitäts- bzw. Abklingzeitmessungen) verwendet werden.

Einige Beispiele:

a) Detektion von Kationen
Erythrosin, Sulforhodamin-B-mono-Natriumsalz, Merocyanine (MC540), Eosin, Phloxine B, 2,6-Dichlorophenolindophenol, Orange IV
b) Detektion von Anionen
3,3'-Diethyloxadicarbocyaninjodid, 1,1'-Diethyl-2,2'-carbocyaninchlorid, 3,3'-Diethylthiacarbocyaninjodide, Quinaldine Red

Beispiele für anionisch solvatochrome Luminophore:
Erythrosin, Sulforhodamin-B-mono-Natriumsalz, Merocyanine (MC540), Eosin, Phloxine B, 2,6-Dichlorophenolindophenol, Orange IV.

Beispiele für kationisch solvatochrome Luminophore:
3,3'-Diethyloxadicarbocyaninjodide, 1,1'-Diethyl-2,2'-carbocyaninchloride, Quinaldine Red

Beschreibung erfindungsgemäß verwendeter Ionophore

Der Ionophor sollte vorzugsweise ungeladen und lipophil sein. Des weiteren soll der Ionophor keine wesentliche pH-Abhängigkeit im Bindungsverhalten aufweisen.

Ionophore für Kationen:
Podanden (offene Kronenether), Coronanden, Cryptanden und Hemicryptanden, Sphäranden und Hemisphäranden, Calixarene und viele weitere, auch "natürliche", wie Valinomycin.

Im speziellen:

| | Bezeichnung | Bezugsquelle | (Bestellnummer) |
|---|---|---|---|
| $Li^+$ | Lithiumionophor I (ETH 149) | Fluka | 62557 |
| | Lithiumionophor II (ETH 1644) | Fluka | 62559 |
| | Lithiumionophor III (ETH 1810) | Fluka | 62561 |
| | Lithiumionophor VI (6,6-Dibenzyl-14-crown-4) | Fluka | 62557 |
| $Na^+$ | Natriumionophor I (ETH 227) | Fluka | 71732 |
| | Natriumionophor II (ETH 157) | Fluka | 71733 |
| | Natriumionophor III (ETH 2120) | Fluka | 71734 |
| | Natriumionophor IV (DD-16-C-5) | Fluka | 71745 |
| | Natriumionophor V (ETH 4120) | Fluka | 71738 |
| | Natriumionophor VI ([Bis-[(12-crown-4)-methyl]-dodecylmethylmalonat] | Fluka | 71739 |
| | Natriumionophor X [4-tert.Butyl-calix[4]aren-tetraessigsäure-tetraethylester] | | |
| | 4-tert.-Butyl-calix[4]aren-tetraessigsäure-tetra(ethylmethylether) ester | Fluka | 71747 |
| K+ | Kaliumionophor I (Valinomycin) | Fluka | 60403 |
| | Kaliumionophor II (Bis[(benzo-15-crown-4)-4'methyl]pimelate) | | |
| | Kaliumionophor III (BME 44) | Fluka | 60397 |
| $Ca^{2+}$ | Calciumionophor I (ETH 1001) | Fluka | 21192 |
| | Calciumionophor II (ETH 129) | Fluka | 21193 |
| | Calciumionophor IV (ETH 5234) | Fluka | 21198 |
| $Mg^{2+}$ | Magnesiumionophore I (ETH 1117) | Fluka | 63082 |
| | Magnesiumionophore II (ETH 5214) | Fluka | 63083 |
| | Magnesiumionophore III (ETH 4030) | Fluka | 63086 |
| | Magnesiumionophore IV (ETH 7025) | Fluka | 63088 |
| | Magnesiumionophore V (K22B1B5) | Fluka | 63103 |

Ionophore für Anionen:
Nonactin, cyclische Lewissäuren, usw.
Im speziellen:

| | | | |
|---|---|---|---|
| Cl | Chlorid Ionophore II (ETH 9009) | Fluka | 24901 |
| | Chlorid Ionophore III (ETH 9033) | Fluka | 24894 |

Beschreibung erfindungsgemäß verwendeter Polymere und co-Polymere:

Typische Materialien zur Verwendung in der Ausführungsform gemäß Fig. 1 sind hydrophile Polymere, welche im Gleichgewicht mit entionisiertem Wasser einen Wassergehalt von >10%, vorzugsweise >30% aufweisen. Einige typische Materialien sind z.B. Polyacrylamide, Poly-HEMA, Polyurethane, Gelatine, Agarose, Poly(vinylalkohol) und Poly(propylenimin).

Typische Materialien zur Verwendung in der Ausführungsform gemäß Fig. 2 sind hydrophile Multi-block-copolymere, welche im Gleichgewicht mit entionisiertem Wasser einen Wassergehalt von >10%, vorzugsweise >30% aufweisen, mit abwechselnden Kettensequenzen aus hydrophilen Gruppen und lipophilen Gruppen bzw. Gruppen mit hoher cohäsiver Energie. Typische Materialien sind z.B. HYPAN-Hydrogele.

Beschreibung erfindungsgemäß verwendeter Weichmacher

Weichmacher sind typischerweise hochsiedende (>150°C), lipophile, polare Flüssigkeiten. Wie in den nachfolgenden Beispielen gezeigt wird, sind geeignete Weichmacher z.B. 2-Cyanophenyldodecylether (CPDDE) und Tris (ethylhexyl)phosphat (TOP). Andere geeignete Weichmacher sind z.B. 2-Cyanophenyloctylether (CPOE), Triresylphosphat, Dioctylphthalat, Tris-2-ethylhexylphosphat, Di-2-ethylhexylsebacat, n-Butylacetyl-ricinoleat, Nitrophenylether, wie z.B. 2-Nitrophenyloctylether (NPOE), 2-Nitrophenylbutylether, Debenzylether, and o-Nitrophenyl-2-(1,3,3)-trimethyl-butyl-5,7,7-triethyloctylether.

Beschreibung erfindungsgemäß verwendeter Tenside

Tenside sind dadurch gekennzeichnet, daß sie sowohl mindestens einen hydrophilen als auch mindestens einen lipophilen Bereich aufweisen. Vorzugsweise tragen die hydrophilen Bereiche keine Ladung und ändern ihre Hydrophilie nicht in Abhängigkeit vom pH. Beispiele für derartige Substanzen sind teilweise mit langkettigen Fettsäuren veresterte Polyalkohole, Glycerine, Glucoside, Phospholide oder auch langkettige Alkohole.

Beispiele:

ISOLAN GI34 (Triglycerin-4-isostearat) und TEGO-CARE 450 (Stearylglucosid) der Fa. Th. Goldschmidt AG, Essen; Triton CF10, Triton N-101, Triton X-45, Triton X-100, Brij 35, Brij 56, Tween 20, Tween 40, Tween 60 und Tween 80 der Fa. Fluka, Deutschland.

Mit den nachfolgenden Beispielen wird die Herstellung bevorzugter Ausführungsformen von erfindungsgemäßen Sensoren beschrieben, wobei die Beispiele 1 bis 4 die Ausführungsform gemäß Fig. 2c betreffen und die Beispiele 5 und 6 die Ausführungsform gemäß Fig. 1 betreffen.

Beispiel 1 (Natrium-Sensor)

100 mg Hydrogel HN80 wurden in 2 ml DMSO durch Anwendung von Ultraschall (12 h; 40°C) gelöst. Dieser Lösung wurden 15 mg CPDDE zugegeben. 9 mg 4-tert.Butylcalix[4]aren-tetraessigsäure-tetra(ethylmethylether)ester wurden zugegeben. 250 µl einer solchen Lösung wurden auf eine Polyesterscheibe mit einem Durchmesser von 24 mm gegossen. Dann wurde die Scheibe 12 h einer mit Wasserdampf gesättigten Atmosphäre ausgesetzt, wobei sie Wasserdampf aufnahm und dabei trüb wurde. Anschließend wurde die Scheibe zur Entfernung von DMSO gründlich mit Wasser gespült und für 24 h in 10 ml einer wässerigen Lösung enthaltend 0,11 mg Merocyanin 540 gegeben. Nach gründlichem Spülen mit Wasser war die Scheibe als Natrium-Sensor für die unten beschriebene Messung bereit. Das Ergebnis der Messung ist in der Fig. 4 dargestellt.

Beispiel 2 (Kalium-Sensor)

100 mg Hydrogel HN80 wurden in 2 ml DMSO durch Anwendung von Ultraschall (12 h; 40°C) gelöst Dieser

Lösung wurden 15 mg CPDDE zugegeben. 2 mg Valinomycin wurden zugegeben. 250 µl einer solchen Lösung wurden auf eine Polyesterscheibe mit einem Durchmesser von 24 mm gegossen. Dann wurde die Scheibe 12 h einer mit Wasserdampf gesättigten Atmosphäre ausgesetzt, wobei sie Wasserdampf aufnahm und dabei trüb wurde. Anschließend wurde die Scheibe zur Entfernung von DMSO gründlich mit Wasser gespült und für 24 h in 10 ml einer wässerigen Lösung enthaltend 0,11 mg Merocyanin 540 gegeben (A.S. Waggoner. Annu. Rev. Biophys. Bioeng. 8, 47 (1979); "Mechanism of the Membrane Potential Sensitivity of the Fluorescent Membrane Probe Merocyanine 540", P.R. Dragston, W.W. Webb. Biochemistry 17,5228 (1978); "pH Dependence of Merocyanine 450 Absorption and Fluorescence Spectra", B.Cunderlikova, Spectrochim. Acta 53A, 293 (1977); "Merocyanine 540 - A Fluorescent Dye and a Biological Probe", C.Parkany, A. Adenier, J.-J. Aaron. NATO ASI Ser, Ser A, S. 286 (1986)). Nach gründlichem Spülen mit Wasser war die Scheibe als Kaluim-Sensor für die unten beschriebene Messung bereit. Das Ergebnis der Messung ist in der Fig. 5 dargestellt.

Beispiel 3 (Kalzium-Sensor)

100 mg Hydrogel HN80 wurden in 2 ml DMSO durch Anwendung von Ultraschall (12 h; 40°C) gelöst. Dieser Lösung wurden 15 mg CPDDE zugegeben. 0,35 mg ETH129 wurden zugegeben. 250 µl einer solchen Lösung wurden auf eine Polyesterscheibe mit einem Durchmesser von 24 mm gegossen. Dann wurde die Scheibe 12 h einer mit Wasserdampf gesättigten Atmosphäre ausgesetzt, wobei sie Wasserdampf aufnahm und dabei trüb wurde. Anschließend wurde die Scheibe zur Entfernung von DMSO gründlich mit Wasser gespült und für 24 h in 10 ml einer wässerigen Lösung enthaltend 0,011 mg Merocyanin 540 gegeben. Nach gründlichem Spülen mit Wasser war die Scheibe als Kalzium-Sensor für die unten beschriebene Messung bereit. Das Ergebnis der Messung ist in der Fig. 6 dargestellt.

Es hat sich gezeigt, daß das Ansprechen des Sensors verbessert werden kann, wenn er 30 min mit einem Kalzium-freien Puffer (20 mMol MOPS/MOPSNa; pH 7,2; Ionenstärke 160 mMol, eingestellt mit NaCl) behandelt wird.

Beispiel 4 (Chlorid-Sensor)

200 mg Hydrogel HN80 wurden in 5 ml DMSO durch Anwenden von Ultraschall (12h; 60°C) gelöst. Dieser Lösung wurden 35,8 mg CPDDE und 2,4 mg ETH9009 (für Chlorid selektiver Ionophor) zugegeben. 200 µl einer solchen Lösung wurden auf eine Polyesterscheibe mit einem Durchmesser von 24 mm gegossen. Dann wurde die Scheibe 12 h einer mit Wasserdampf gesättigten Atmosphäre ausgesetzt, wobei sie Wasserdampf aufnahm und dabei trüb wurde. Anschließend wurde die Scheibe zur Entfernung von DMSO gründlich mit Wasser gespült und für 24 h in 10 ml einer wässerigen Lösung enthaltend 0,01 mg 3,3'-Diethyloxadicarbocyaninjodid (Fluka Nr. 32446) gegeben. Das Ergebnis der Messung ist in der Fig. 7 dargestellt.

Beispiel 5 (Kalium-Sensor)

Es wurde eine Emulsion von Trioctylphosphat hergestellt, indem 5 mg Trioctylphosphat, 5 mg Isolan GI 34 und 1 mg Valinomycin in 1 ml Ethanol aufgelöst wurden. Unter starkem Rühren wurden 2 ml heißes Wasser langsam zugegeben. Nach Abdampfen des Ethanols bei vermindertem Druck wurden 100 mg Cyanogum 41 (Serva), 5 mg $Na_2SO_3$, 50 µl Polymerisationskatalysator (DMAPN; Serva) und 50 µl einer 5%igen (Gew./Gew.) wässerigen Lösung von Kaliumperoxodisulfat zugegeben. 300 µl dieses Gemisches wurden auf eine Glasplatte mit einem Durchmesser von 25 mm gegossen und in einer Stickstoff-Atmosphäre gelassen, bis die Polymerisation beendet war (etwa 30 min). Nach gründlichem Spülen mit Wasser wurde die Membran 24 h in 10 ml einer wässerigen Lösung enthaltend 0,011 mg Merocyanin 540 gegeben. Das Ergebnis der Messung ist in der Fig. 8 dargestellt.

Beispiel 6 (Chlorid-Sensor)

11 mg eines Gemisches (1:1) von Isolan GI 34, einem flüssigen Triglycerin-4-isostearat (Thomas Goldschmidt AG; Essen) und Tris(ethylhexyl)phosphat wurden zusammen mit 1 mg ETH 9009 (Chlorid-Ionophor; Fluka) in 2 ml Ethanol gelöst. Das Gemisch wurde stark gerührt und 2 ml heißes Wasser wurden während des Rührens langsam zugegeben, worauf weitere 10 min gerührt wurde. Das Gemisch wurde im Exsikkator bei vermindertem Druck eine weitere Stunde aufbewahrt, um Ethynol und Sauerstoff zu entfernen. Danach wurden 105 mg Cyanogum 41, 5 mg $Na_2SO_3$, 25 µl Dimethylaminopropionitril (DMAPN; Fluka) als Katalysator und 25 µl wässerige Lösung von Peroxodisulfat (2%; Gew./Gew.) als Starter zugegeben.

300 µl dieses Gemisches wurden auf einmal in einer Stickstoff-Atmosphäre auf eine Glasplatte getropft. Nach 5 min war die Polymerisation beendet. Die Glasplatte wurde gründlich mit Wasser gewaschen und danach mit einer 0,025 mg in 10 ml Lösung von 3,3'-Diethyloxadicarbocyaninjodid behandelt. Das Ergebnis der Messung ist in der Fig. 9 dargestellt.

Beschreibung der Messungen und Meßapparatur

Zur Bestimmung der Lumineszenzintensität in Abhängigkeit der Meßgröße (cNa$^+$, cK$^+$, cCa$^{++}$, cCl$^-$) wurden die verschiedenen Sensorscheiben in eine Durchflußzelle mit einer für Anregungs- und Lumineszenzlicht durchlässigen Öffnung eingebracht, wobei die beschichtete Seite der Scheiben einen Teil des Probenkanals der Durchflußzelle bildete, während die nichtbeschichtete Seite die lichtdurchlässige Öffnung der Durchflußzelle verschloß.

Zur Bestimmung der Abhängigkeit der rel. Lumineszenzintensität der verschiedenen Sensoren in Abhängigkeit ihrer Meßgröße (cNa$^+$, cK$^+$, cCa$^{++}$ bzw. cCl$^-$) wurden die Sensorscheiben in der Durchflußzelle mit Meßflüssigkeiten verschiedener Konzentration an Na$^+$, K$^+$, Ca$^{++}$ bzw. Cl$^-$ bei konstantem pH-Wert in Kontakt gebracht.

Als optisches Meßgerät wurde ein Aminco-Bowman Lumineszenzspektrometer der Serie 2 (SLM-Aminco, Rochester, NY 14625, USA) verwendet. Die Durchflußzelle wurde derart in der Meßkammer des Spektrometers befestigt, daß das Anregungslicht von der im Spektrometer befindlichen Lichtquelle durch den anregungsseitigen Monochromator und weiter in die Durchflußzelle durch das transparente Trägermaterial in die den Ionophor enthaltende Schicht der Sensorscheibe geleitet werden konnte. Ein Teil des vom Farbstoff emittierten Lumineszenzlichtes wurde durch das transparente Trägermaterial zum emissionsseitigen Monochromator und weiter zum Detektor (einem Photomultiplier) geleitet.

Bei sämtlichen Kationensensoren (Na$^+$, K$^+$, Ca$^{++}$) wurde der anregungsseitige Monochromator auf die Wellenlänge 572 nm, und der emissionsseitige Monochromator auf die Wellenlänge 598 nm eingestellt. Bei sämtlichen Anionensensoren (Cl$^-$) wurde der anregungsseitige Monochromator auf die Wellenlänge 595 nm, und der emissionsseitige Monochromator auf die Wellenlänge 626 nm eingestellt.

Vor der Messung wurden die in den Beispielen 1 bis 6 beschriebenen Sensorscheiben bei Raumtemperatur eine halbe Stunde durch Eintauchen in einer Flüssigkeit konditioniert, dann sogleich in die Durchflußzelle eingebracht und die rel. Lumineszenzintensität in Abhängigkeit der Meßgröße (cNa$^+$, cK$^+$, cCa$^{++}$ bzw. cCl$^-$) durch sequentiellen Kontakt der Sensoren mit den unten beschriebenen Meßflüssigkeiten bestimmt.

Beschreibung der Meßflüssigkeiten:

Bei vorgegebener Gesamtkonzentration an pH-Puffersalzen (20mmol/l Phosphat bzw. 20 mmol/l MOPS), vorgegebenem pH-Wert und vorgegebener Ionenstärke wurden die Konzentrationen (c$H_2PO_4^-$ und c$HPO_4^-$ bzw. cMOPS und cMOPS-Na) der herzustellenden Meßflüssigkeiten nach D.D. Perrin, B. Dempsey, "Buffers for pH and Metal Ion Control", Chapman and Hall Laboratory Manuals London, 1974, Kapitel 5, bei einer Temperatur von 20°C berechnet. Die Gesamtionenstärke wurde bei der pH-Wert-Berechnung berücksichtigt. Die Konzentration an Neutralsalzen zwecks Einstellen eines bestimmten Wertes der Meßgröße bzw. der Gesamtionenstärke wurde ebenfalls berechnet.

Zur Herstellung der Meßflüssigkeiten wurden die aus den Konzentrationen berechneten Mengen an Puffer- und Neutralsalzen eingewogen und in einem Maßkolben mit entionisiertem Wasser auf 1 Liter verdünnt.

Die in Fig. 4 (Beispiel 1, Na$^+$) dargestellte Meßkurve wurde durch sequentiellen Kontakt eines Na$^+$ Sensors mit Meßflüssigkeiten konstanten pH-Wertes (7,20) und konstanter Ionenstärke (200 mmol/l) bei 20°C erhalten. Die Meßflüssigkeiten enthielten 5,662 mmol/l $KH_2PO_4$, 14.338 mmol/l $K_2HPO_4$ und 1, 10, 100 bzw. 150 mmol/l NaCl. Die Ionenstärke wurde mit KCl (150,0; 141,3; 51,3 bzw. 1,3 mmol/l) eingestellt. Als Konditionierungsflüssigkeit wurde ein Phosphatpuffer gleicher Zusammensetzung wie oben, jedoch ohne NaCl, verwendet.

Die in den Fig. 5 und 8 (Beispiele 2 und 5, K$^+$) dargestellten Meßkurven wurden durch sequentiellen Kontakt von K$^+$ Sensoren mit Meßflüssigkeiten konstanten pH-Wertes (7,40) und konstanter Ionenstärke (140 mmol/l) bei 20°C erhalten. Die Meßflüssigkeiten enthielten 4,269 mmol/l $KH_2PO_4$, 15,731 mmol/l $K_2HPO_4$ und 0,01; 0,1; 1, 6; 50, 88,5 mmol/l KCl. Die Ionenstärke wurde mit berechneten Mengen an NaCl eingestellt. Als Konditionierungsflüssigkeit wurde ein Phosphatpuffer gleicher Zusammensetzung wie oben, jedoch ohne KCl, verwendet.

Die in Fig. 6 (Beispiel 3, Ca$^{++}$) dargestellte Meßkurve wurde durch sequentiellen Kontakt eines Ca$^{++}$ Sensors mit Meßflüssigkeiten konstanten pH-Wertes (7,20) und konstanter Ionenstärke (160 mmol/l) bei 20°C erhalten. Die Meßflüssigkeiten enthielten 9,0 mmol/l MOPS, 11,0 mmol/l MOPS-Na und 0,01, 0,1, 0,5, 1, 5, 10 bzw. 50 mmol/l $CaCl_2$. Die Ionenstärke wurde mit berechneten Mengen an NaCl eingestellt. Als Konditionierungsflüssigkeit wurde ein MOPS-Puffer gleicher Zusammensetzung wie oben, jedoch ohne $CaCl_2$, verwendet.

Die in den Fig. 7 und 9 (Beispiele 4 und 6, Cl$^-$) dargestellten Meßkurven wurden durch sequentiellen Kontakt von Cl-Sensoren mit Meßflüssigkeiten konstanten pH-Wertes (7,1) und konstanter Ionenstärke (250 mmol/l) bei 20°C erhalten. Die Meßflüssigkeiten enthielten 6,0 mmol/l $NaH_2PO_4$, 14 mmol/l $Na_2HPO_4$ und 1, 10, 50, 100, 150 bzw. 200 mmol/l NaCl. Die Ionenstärke wurde mit berechneten Mengen an $NaNO_3$ eingestellt. Als Konditionierungsflüssigkeit wurde ein Phosphatpuffer gleicher Zusammensetzung wie oben, jedoch ohne NaCl, verwendet.

## Patentansprüche

1. Sensor mit einem hydrophilen Bereich und einem hydrophoben Bereich zum Bestimmen von Ionen in einer wässerigen Probe nach dem Koextraktionsprinzip, gemäß welchem das zu bestimmende Ion zunächst aus der Probe in den hydrophilen Bereich wandert und zusammen mit einem optisch detektierbaren, zum bestimmenden Ion elektrisch gegensinnig geladenen Farbstoff, der im hydrophilen Bereich vorgesehen ist, in den hydrophoben Bereich, der einen Ionophor für das zu bestimmende Ion enthält, extrahiert wird,
   **dadurch gekennzeichnet,**
   **daß** der hydrophobe Bereich im hydrophilen Bereich eingebettet ist.

2. Sensor nach Anspruch 1, **dadurch gekennzeichnet, daß** der hydrophobe Bereich in Form von Tröpfchen im hydrophilen Bereich eingebettet ist.

3. Sensor nach Anspruch 2, **dadurch gekennzeichnet, daß** als Tröpfchen ein Weichmacher, insbesondere eine hochsiedende organische Flüssigkeit, vorgesehen ist.

4. Sensor nach Anspruch 3, **dadurch gekennzeichnet, daß** die Tröpfchen durch ein Tensid stabilisiert sind.

5. Sensor nach Anspruch 3, **dadurch gekennzeichnet, daß** die Tröpfchen durch Kettensequenzen eines Multi-block-co-polymers stabilisiert sind.

6. Sensor nach einem der Ansprüche 1 bis 5 zur Bestimmung von Alkali-, Erdalkali- und Chloridionen.

## Claims

1. A sensor comprising a hydrophilic region and a hydrophobic region for the determination of ions in an aqueous sample according to the principle of coextraction, wherein the ion to be determined at first migrates from the sample into the hydrophilic region and, together with an optically detectable dye electrically charged in opposition to the ion to be determined and provided in the hydrophilic region, is extracted into the hydrophobic region containing an ionophore for the ion to be determined,
   **characterized in that**
   the hydrophobic region is embedded in the hydrophilic region.

2. A sensor according to claim 1, **characterized in that** the hydrophobic region is embedded in the hydrophilic region in the form of droplets.

3. A sensor according to claim 2, **characterized in that** as droplets a plasticizer, in particular a high-boiling organic liquid, is provided.

4. A sensor according to claim 3, **characterized in that** the droplets are stabilized by means of a tenside.

5. A sensor according to claim 3, **characterized in that** the droplets are stabilized by means of chain sequences of a multi-block-co-polymer.

6. A sensor according to any one of claims 1 to 5 for the determination of alkali, alkaline-earth and chloride ions.

## Revendications

1. Détecteur avec un domaine hydrophile et un domaine hydrophobe pour le dosage d'ions dans un échantillon aqueux selon le principe de co-extraction, selon lequel l'ion à doser est d'abord transporté hors de l'échantillon dans le domaine hydrophile et, avec un colorant détectable optiquement chargé dans le sens contraire électriquement à l'ion à doser qui est prévu dans le domaine hydrophobe, est extrait dans le domaine hydrophobe qui contient un ionophore pour l'ion à doser, **caractérisé en ce que** le domaine hydrophobe est enrobé dans le domaine hydrophile.

2. Détecteur selon la revendication 1, **caractérisé en ce que** le domaine hydrophobe est enrobé dans le domaine

hydrophile sous forme de gouttelettes.

3. Détecteur selon la revendication 2, **caractérisé en ce que** comme gouttelettes on prévoit un plastifiant, en particulier un liquide organique à point d'ébullition élevé.

4. Détecteur selon la revendication 3, **caractérisé en ce que** les gouttelettes sont stabilisées par un tensioactif.

5. Détecteur selon la revendication 3, **caractérisé en ce que** les gouttelettes sont stabilisées par des séquences de chaînes d'un copolymère à séquences multiples.

6. Détecteur selon l'une des revendications 1 à 5 pour le dosage d'ions alcalins, alcalino-terreux et chlorures.

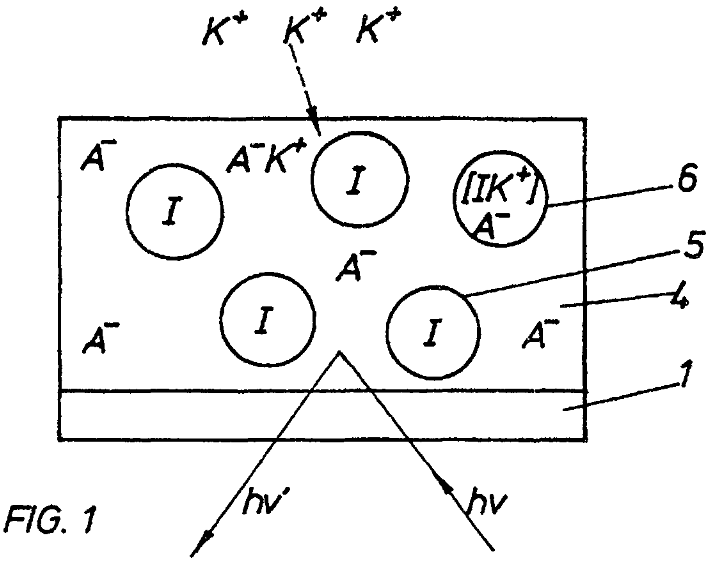


FIG. 1

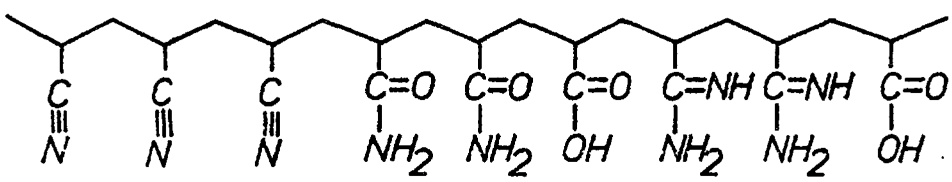

FIG. 2a

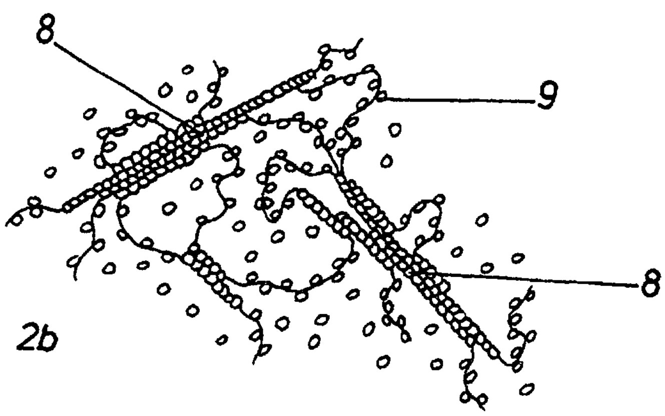


FIG. 2b

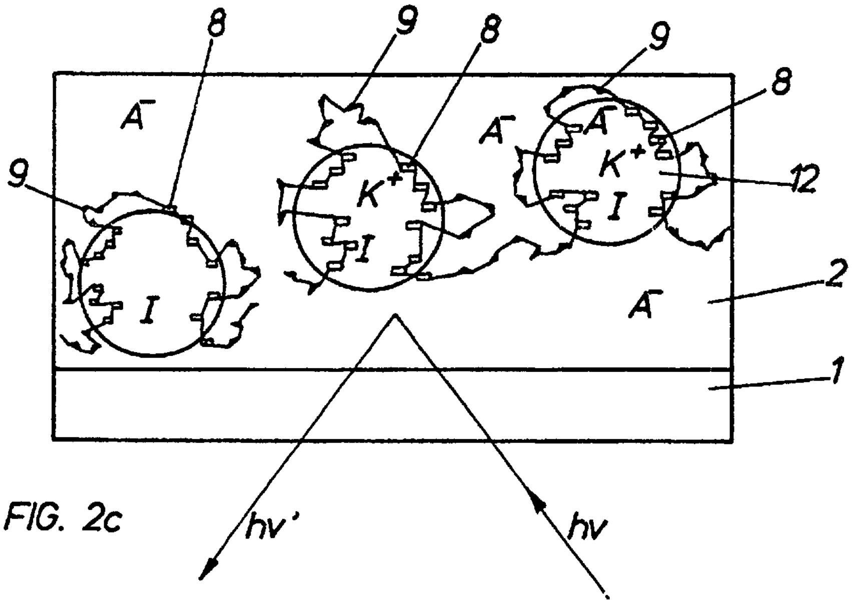


FIG. 2c

FIG. 3

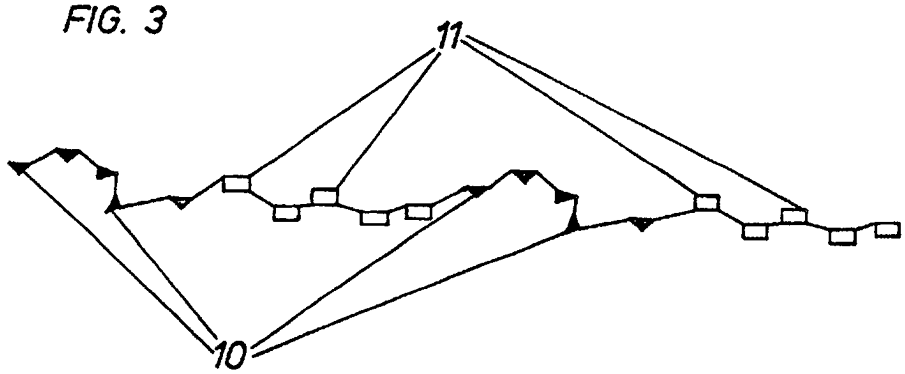

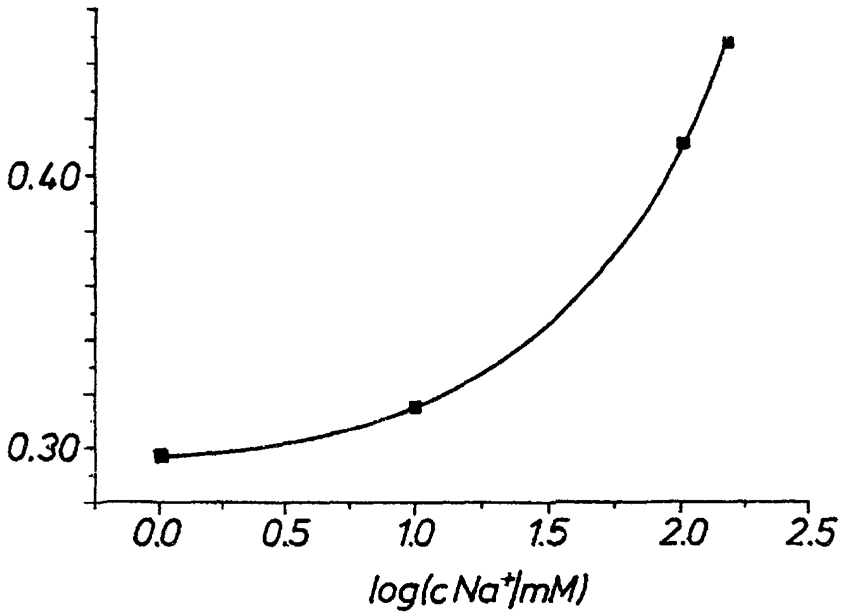


FIG. 4

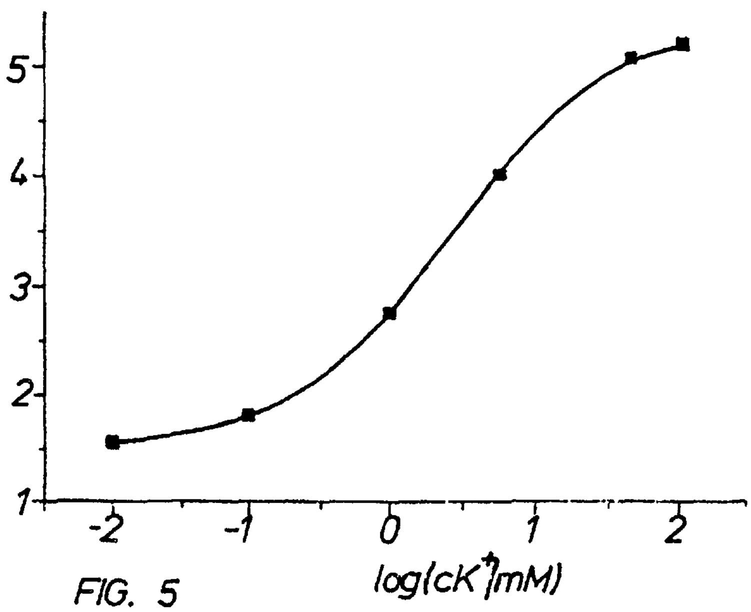


FIG. 5

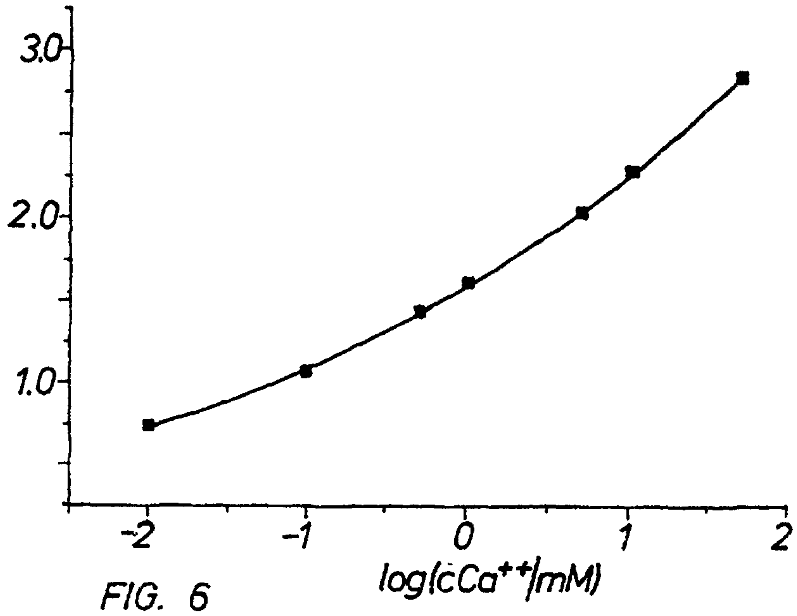
3.0
2.0
1.0
-2
-1
0
1
2
log(cCa++/mM)

FIG. 6

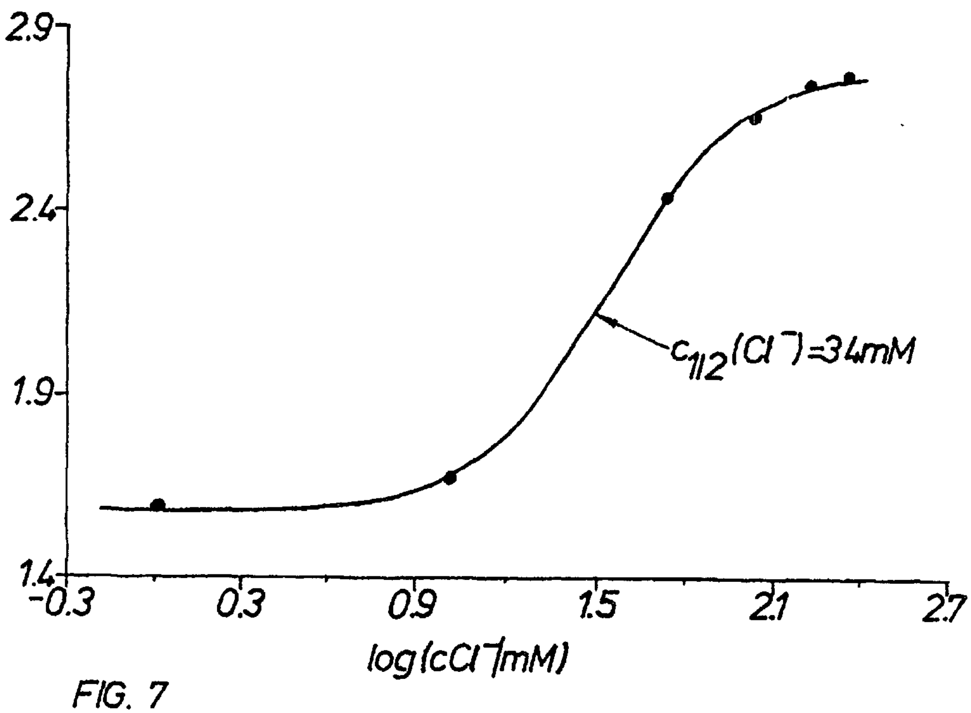
2.9
2.4
1.9
1.4
-0.3
0.3
0.9
1.5
2.1
2.7
c1/2(Cl−)=34mM
log(cCl−/mM)

FIG. 7

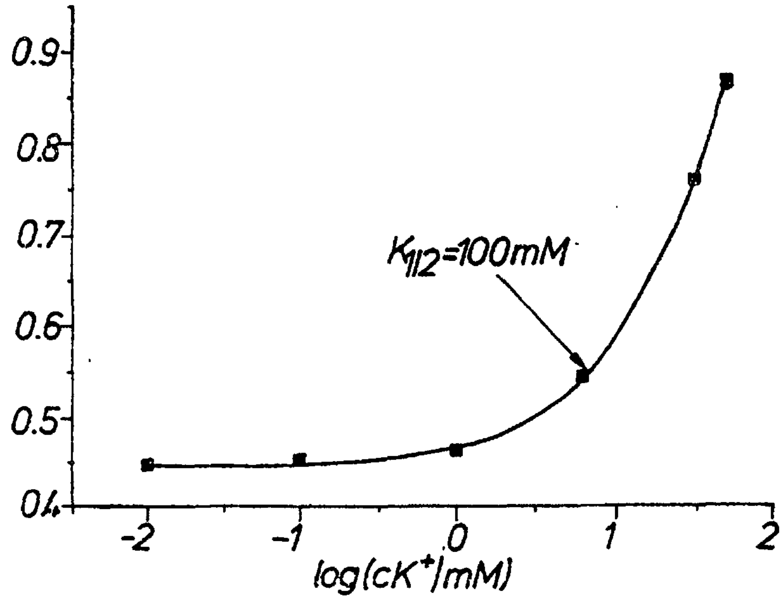
0.9
0.8
0.7
0.6
0.5
0.4
-2
-1
0
1
2
log(cK+/mM)
K1/2=100mM

FIG. 8

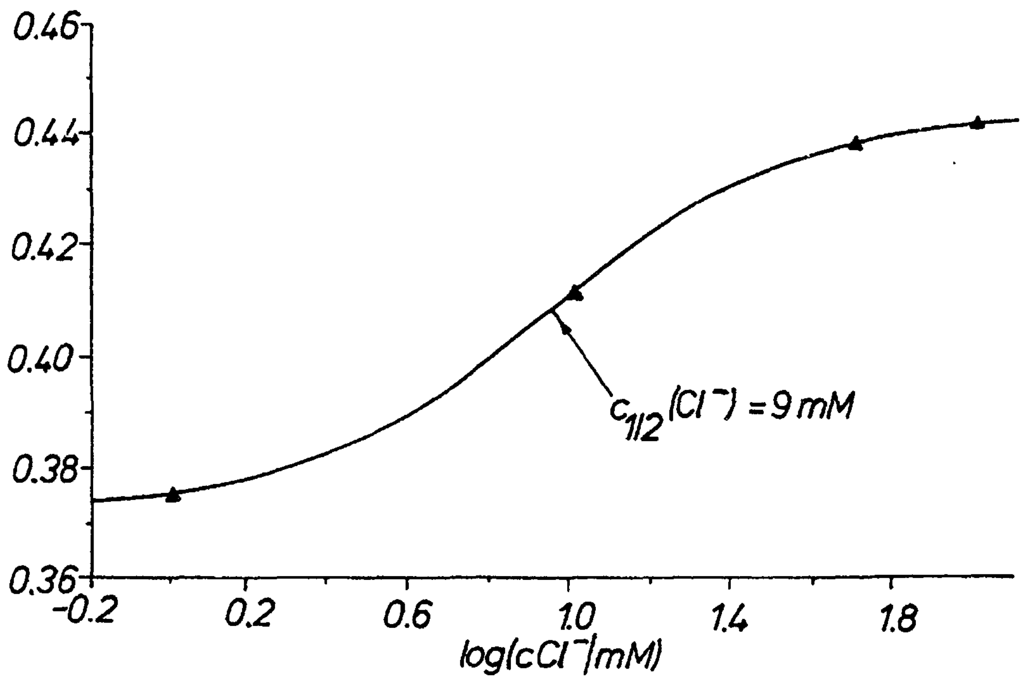
0.46
0.44
0.42
0.40
0.38
0.36
-0.2
0.2
0.6
1.0
1.4
1.8
log(cCl-/mM)
c1/2(Cl-) = 9 mM

FIG. 9